# EUROPEAN PATENT SPECIFICATION

(11) **EP 4 076 300 B1**
(45) Date of publication and mention of the grant of the patent: **20.03.2024**
(21) Application number: 20838190.5
(22) Date of filing: 17.12.2020
(51) Int. Cl.: A61F 5/445, A61F 5/449, A44B 11/25

(54) **SYSTEM COMPRISING A HOOK AND A BELT MOUNT FOR OSTOMY APPLIANCES**
SYSTEM MIT EINEM HAKEN UND EINER GÜRTELHALTERUNG FÜR STOMAVORRICHTUNGEN
SYSTÈME COMPRENANT UN CROCHET ET UN SUPPORT DE CEINTURE POUR LES APPAREILLAGES DE STOMIE

(30) Priority: 19.12.2019 GB 201918857; 19.12.2019 GB 201918854; 19.12.2019 GB 201918869
(43) Date of publication of application: 26.10.2022
(73) Proprietor: ConvaTec Limited, Deeside, Flintshire CH5 2NU (GB)
(72) Inventor: TAAL, Stefan, London W6 7HJ (GB); HOLROYD, Simon, London W6 7HJ (GB); BAKER, Dominic, London W6 7HJ (GB)
(74) Representative: Wilson Gunn
(86) International application number: PCT/GB2020/053258
(87) International publication number: WO 2021/123786

(56) References cited:
- EP-A2- 1 008 313
- FR-A1- 2 948 856
- GB-A- 1 212 904
- US-A- 3 074 404
- US-A- 3 789 846
- US-A1- 2015 359 658
- US-B1- 7 496 994

## Description

### Technical Field

The present disclosure relates to the field of ostomy appliances for managing effluent from a stoma and relates in particular to a system comprising a hook and a belt mount for attaching a belt to a wafer of an ostomy appliance.

### Background of the disclosure

There are many forms of ostomy appliance which try to provide a secure, comfortable fit for ostomates.

US8945076 describes a one-piece ostomy appliance adapted to be removably attached to a patient for receiving discharge from a stoma of the patient that includes a pouch that defines a cavity. A first opening is defined by the pouch for receiving a stoma and communicating discharge from the stoma into the cavity. The ostomy appliance also includes a face plate having a first side secured to the pouch and a second opposing side that includes an adhesive for securing the pouch to a patient. A second opening is defined by the face plate and is generally aligned with the first opening. Further, the ostomy appliance includes one or more tabs for coupling a belt thereto, wherein the one or more tabs are coupled directly to the face plate. The one or more tabs allow the pouch to be secured to a patient by a belt or other similar mechanism.

GB1212904A discloses an ileostomy appliance attached to a patient by two elasticated belts comprising clips and discloses the features of the preamble of claim 1. The clips engage with lugs on a pressure plate of the ileostomy appliance.

EP1008313A2 discloses a snap-in locking device for securing holding elements for objects such as flashlights, mobile phones, pistols or similar equipment to clothing. The device has an anchor plate that engages a slide member. The anchor plate comprises a tongue which limits the movement of the slide member to prevent unintentional separation of the slide member from the anchor plate.

FR2948856A1 discloses a device for fixing an accessory (e.g. a handgun) to the thigh of a user. The device comprises two interlocking elements, a first element comprising a plate with a T-shaped groove and a second element comprising a plate with a tab that is moveable within the T-shaped groove to lock the elements together. The first element also comprises a cover over one end of the groove to assist guidance of the tab into the groove.

The present applicant has found that ostomates may find it difficult to secure an ostomy appliance to their body. This can also be the case with ostomy appliances that are provided with one or more tabs for securing to a belt or other similar mechanism. In particular, the ostomate may have difficulty in locating the one or more tabs (which may be obscured from sight during mounting by other parts of the ostomy appliance). Further, they may have difficulty in physically attaching the one or more tabs (this difficulty which may be exacerbated if the ostomate has reduced manual dexterity). Further, the process of attaching the one or more tabs to the belt or similar mechanism may not engender a feeling of security in the ostomate if they find it difficult to establish if a good connection between the one or more tabs and the belt or similar mechanism has been achieved. Further, they may face difficulties in easily detaching the one or more tabs from the belt or similar mechanism when wishing to empty or replace the ostomy appliance (this difficulty may also be exacerbated if the ostomate has reduced manual dexterity).

In light of this, there remains a need for a belt mount for ostomy appliances with enhanced usability for ostomates, particularly in the areas of ease of use and security of use.

### Summary of the disclosure

In this specification, the term "stomal output" refers to any gases or fluids or solids produced by an ostomate that may be secreted from the stoma or that exit the stoma. The stomal output may comprise stomal gas, stomal liquid and stomal solids.

In this specification, the term "stoma" refers to an opening in the body. Generally the stoma is a surgical opening in the torso of the body. In some instances, the term "stoma" also refers to internal tissue, organs or portions thereof that are exposed by the opening. By way of non-limiting example, internal tissue may be selected from colon, ileum, small intestine, large intestine, jejunum, and duodenum, and combinations thereof. The internal tissue may be an end or a loop of a small or large intestine.

In this specification, the term "ostomate" refers to a subject that may have use of the belt mount for an ostomy appliance disclosed herein. While ostomate usually refers to a subject with a surgical opening, as used herein, "ostomate" may refer to a subject who has a stoma, regardless of whether the stoma was created by surgery or other means.

The term "user" may refer to an ostomate, or to another person assisting the ostomate, for example, with emptying of the stomal output from the cavity.

In this specification, the ostomy appliances disclosed herein may, for example, be used for managing a stoma created by an esophagostomy, a gastrostomy, a cholecystostomy, a choledochostomy, a cecostomy, a colostomy, a duodenostomy, an ileostomy, a jejunostomy, an appendicostomy, a tracheostomy, a urostomy, a nephrostomy, an ureterostomy, or a vesicostomy. The ostomy appliances disclosed herein may be used with additional devices including, but not limited to, a shunt, a catheter, a plug or a fecal management system.

In this specification locations and orientations of features may be described with reference to the belt mount for an ostomy appliance being "in use", "orientated as it would be in use" or similar. Such terms refer to the intended orientation of the belt mount for an ostomy appliance when it is secured to a body of an ostomate with the ostomate in a standing position, irrespective of whether the ostomy appliance is currently performing such a use or the actual position of the ostomate. The terms "upper" and "lower" and related terms refer to the relative position of a part or portion of the belt mount for an ostomy appliance when orientated as it would be in use.

Ostomy appliances are commonly attached to the body by means of a wafer which includes an adhesive layer or layers. The wafer typically comprises an adhesive layer on a body-facing side for adhering the wafer to the body of the ostomate. Typically, a release liner covers a body-facing side of the wafer that is removed by the user prior to fitting to the skin. In this specification, the term "wafer" may be used interchangeably with the terms "adapter," "ostomy wafer," "baseplate", or "layered adhesive wafer." In this specification, the term "wafer" includes wafers for a "two-piece appliance" and for a "one-piece appliance".

In this specification a "two-piece appliance" refers to an appliance where the wafer forms part of a separate body fitment component that is attached by a releasable coupling to a pouch appliance. A two-piece appliance permits the body fitment component to be separated from the pouch appliance without damage, so that at least one of the parts continues to be functionally usable. For example, the body fitment component may remain in place on the body of the ostomate. In this specification a "one-piece appliance" refers to an appliance where the wafer is permanently attached to the appliance, to the extent that the wafer cannot easily be separated without risk of damaging the appliance. A one-piece appliance is intended to be used as an integral unit. In this specification, the belt mounts for ostomy appliances disclosed herein may, for example, be used with one-piece and two-piece appliances.

According to an aspect of the invention there is provided a system according to claim 1.

Additionally or alternatively, the tongue extends in a plane that is parallel with a face of the mounting ring. The tongue also extends from a proximal end of the mounting aperture. The tongue has a concave end that is contactable with the hook if the mounting ring and the hook are pushed towards each other.

Additionally or alternatively, the belt mount has a clearance either side of tongue and in between the tongue and the mounting ring, and the tongue is cantilevered from a supporting portion of the belt mount. The tongue is flexible. More specifically the tongue is configured to flex and ride over a back portion of the hook on insertion of the portion of the mounting ring into the mouth of the hook.

Additionally or alternatively, the belt mount also has at least one protrusion that extends from at least one of a first face and a second face of the mounting ring to contact the hook when inserting the portion of the mounting ring into the mouth of the hook. The at least one protrusion is located at a distal end of the mounting ring opposite the tongue. The at least one protrusion is located adjacent an inner edge of the mounting ring. Further, the at least one protrusion has a ramped surface facing towards the mounting aperture. The at least one protrusion has a width no greater than a width of the tongue.

Additionally or alternatively a width of the cantilevered hook element is less than a width of the mounting aperture so as to accommodate limited rotation of the hook and the baseplate about an axis extending perpendicularly to a plane of the mounting ring. The limited rotation of the hook and the baseplate is guided by contact between a concave end of the tongue and a convex face of the cantilevered hook element. Further, the limited rotation of the hook and the baseplate is constrained by contact between the cantilevered hook element and the mounting ring.

Additionally or alternatively, the hook comprises a baseplate and the tongue is configured to contact the baseplate to cause at least one of a tactile feedback and an audible feedback as the tongue recovers its shape upon successful insertion of the portion of the mounting ring into the mouth of the hook.

Another embodiment the mounting ring may have at least one protrusion that extends from at least one of a first face and a second face of the mounting ring to contact the hook when inserting the portion of the mounting ring into the mouth of the hook.

Additionally or alternatively, at least one protrusion is configured to provide a resistance to insertion of the portion of the mounting ring into the mouth of the hook.

Additionally or alternatively, overcoming the resistance to insertion provided by the at least one protrusion is configured to produce at least one of a tactile feedback and an audible feedback signaling successful insertion of the portion of the mounting ring into the mouth of the hook.

Additionally or alternatively, the mounting ring at least partially surrounds a mounting aperture.

Additionally or alternatively, the at least one protrusion is located at a distal end of the mounting aperture.

Additionally or alternatively, the at least one protrusion is located adjacent an inner edge of the mounting ring.

Additionally or alternatively, the at least one protrusion comprises a ramped surface facing towards the mounting aperture.

Additionally or alternatively, the at least one protrusion has a width no greater than a width of the hook.

Additionally or alternatively, the at least one protrusion is formed from a compressible material, preferably a resilient material.

Additionally or alternatively, only a single protrusion extends from the first face of the mounting ring and only a single protrusion extends from the second face of the mounting ring.

Additionally or alternatively, both single protrusions are aligned with one another about a circumference of the mounting ring.

Additionally or alternatively, the hook comprises a baseplate and a cantilevered hook element, wherein a mouth of the hook is defined between the baseplate and the cantilevered hook element.

Additionally or alternatively, the at least one protrusion is configured to be received in an aperture in the baseplate on insertion of the portion of the mounting ring into the mouth of the hook.

Additionally or alternatively, the at least one protrusion received in the aperture in the baseplate is a protrusion that extends from the second face of the mounting ring.

Additionally or alternatively, a recess is on an underside of the cantilevered hook element, wherein the at least one protrusion is configured to be received in the recess on insertion of the portion of the mounting ring into the mouth of the hook.

Additionally or alternatively, the at least one protrusion received in the recess of the cantilevered hook element is a protrusion that extends from the first face of the mounting ring.

Additionally or alternatively, a clearance of the mouth between the baseplate and the cantilevered hook element is less in dimension than a total thickness of the mounting ring and the at least one protrusion such that insertion of the portion of the mounting ring into the mouth of the hook must be accommodated by one or more of deflection of the cantilevered hook element and compression of the at least one protrusion.

Additionally or alternatively, the mounting ring at least partially surrounds a mounting aperture and the mounting ring comprises at least one chamfered edge.

Additionally or alternatively, the at least one protrusion is located adjacent to the chamfered edge of the mounting ring.

Additionally or alternatively, the at least one protrusion comprises a ramped surface facing towards the mounting aperture.

Additionally or alternatively, wherein the ramped surface is formed as a smooth continuation of the chamfered edge.

Additionally or alternatively, the mounting ring may at least partially surround a mounting aperture which accommodates at least a portion of the hook upon successful insertion of the portion of the mounting ring into the mouth of the hook. Further, the mounting ring bordering the mounting aperture may have at least one chamfered edge.

Additionally or alternatively, the chamfered edge is on a first face of the mounting ring.

Additionally or alternatively, the chamfered edge comprises a U-shaped chamfered edge.

Additionally or alternatively, at least one protrusion that extends from at least one of a first face and a second face of the mounting ring to contact the hook when inserting the portion of the mounting ring into the mouth of the hook.

Additionally or alternatively, the at least one protrusion is located adjacent to the chamfered edge of the mounting ring.

Additionally or alternatively, the at least one protrusion comprises a ramped surface facing towards the mounting aperture.

Additionally or alternatively, the ramped surface is formed as a smooth continuation of the chamfered edge.

Additionally or alternatively, the hook comprises a baseplate and a cantilevered hook element, wherein a mouth of the hook is defined between the baseplate and the cantilevered hook element.

Additionally or alternatively, a clearance of the mouth between the baseplate and the cantilevered hook element is greater in dimension than a thickness of the chamfered edge of the mounting ring.

The at least one protrusion may be configured to provide a resistance to insertion of the portion of the mounting ring into the mouth of the hook. Overcoming the resistance to insertion provided by the at least one protrusion may be configured to produce at least one of a tactile feedback and an audible feedback signalling successful insertion of the portion of the mounting ring into the mouth of the hook.

The mounting ring may at least partially surround a mounting aperture. The at least one protrusion may be located at a distal end of the mounting aperture. The at least one protrusion may be located adjacent an inner edge of the mounting ring. The at least one protrusion may comprise a ramped surface facing towards the mounting aperture.

The at least one protrusion may have a width no greater than a width of the hook. The at least one protrusion may be formed from a compressible material, preferably a resilient material.

The belt mount may comprise only a single protrusion that extends from the first face of the mounting ring and only a single protrusion that extends from the second face of the mounting ring. Both single protrusions may be aligned with one another about a circumference of the mounting ring.

The at least one protrusion may be configured to be received in an aperture in the baseplate on insertion of the portion of the mounting ring into the mouth of the hook. The at least one protrusion received in the aperture in the baseplate may be a protrusion that extends from the second face of the mounting ring.

The system may further comprise a recess on an underside of the hook element, wherein the at least one protrusion may be configured to be received in the recess on insertion of the portion of the mounting ring into the mouth of the hook. The at least one protrusion received in the recess of the hook element may be a protrusion that extends from the first face of the mounting ring.

A clearance of the mouth between the baseplate and the cantilevered hook element may be less in dimension than a total thickness of the mounting ring and the at least one projection such that insertion of the portion of the mounting ring into the mouth of the hook must be accommodated by one or more of deflection of the cantilevered hook element and compression of the at least one projection.

The tongue may extend in a plane of the mounting ring. The tongue may extend from a proximal end of the mounting aperture. The tongue may comprise a concave end that is contactable with the hook if the mounting ring and the hook are pushed towards each other.

The belt mount may further comprise a clearance either side of tongue and in between the tongue and the mounting ring, and optionally the tongue may be cantilevered from a supporting portion of the belt mount.

The tongue may be flexible. The tongue may be configured to flex and ride over a back portion of the hook on insertion of the portion of the mounting ring into the mouth of the hook.

The belt mount may further comprise at least one protrusion that extends from at least one of a first face and a second face of the mounting ring to contact the hook when inserting the portion of the mounting ring into the mouth of the hook. The at least one protrusion may be located at a distal end of the mounting ring opposite the tongue. The at least one protrusion may be located adjacent an inner edge of the mounting ring. The at least one protrusion may comprise a ramped surface facing towards the mounting aperture. The at least one protrusion may have a width no greater than a width of the tongue.

A width of the cantilevered hook element may be less than a width of the mounting aperture so as to accommodate limited rotation of the hook and the baseplate about an axis extending perpendicularly to a plane of the mounting ring. The limited rotation of the hook and the baseplate may be guided by contact between a concave end of the tongue and a convex face of the cantilevered hook element. Optionally, the limited rotation of the hook and the baseplate may be constrained by contact between the cantilevered hook element and the mounting ring.

The mounting ring bordering the mounting aperture may comprise at least one chamfered edge.

The chamfered edge may be on a first face of the mounting ring. The chamfered edge may comprise a U-shaped chamfered edge.

The belt mount may further comprise at least one protrusion that extends from at least one of a first face and a second face of the mounting ring to contact the hook when inserting the portion of the mounting ring into the mouth of the hook. The at least one protrusion may be located adjacent to the chamfered edge of the mounting ring. The at least one protrusion may comprise a ramped surface facing towards the mounting aperture. The ramped surface may be formed as a smooth continuation of the chamfered edge.

A clearance of the mouth between the baseplate and the cantilevered hook element may be greater in dimension than a thickness of the chamfered edge of the mounting ring.

### Brief description of the drawings

One or more embodiments of the disclosure will now be described, by way of example only, with reference to the accompanying drawings in which:
Figure 1 illustrates a schematic representation of an ostomy appliance that comprises a wafer;
Figure 2 is a top view of a wafer that comprises a belt mount having two mounting rings;
Figure 3 is an enlarged view of a portion of the wafer of Figure 2 showing one of the mounting rings;
Figure 4 is a perspective view from above of the wafer of Figure 2;
Figure 5 is a cross-sectional view taken on line V-V of Figure 3;
Figure 6 is a perspective view from above of one of the mounting rings of the wafer of Figure 2;
Figure 7 illustrates a belt which comprises a hook at each end;
Figure 8 illustrates a hook of the belt of Figure 7 attached to one of mounting rings of the wafer of Figure 2;
Figure 9 illustrates the hook and belt mount of Figure 8 during the process of inserting the mounting ring into the hook; and
Figure 10 illustrates the hook and belt mount of Figure 8 during the process of detaching the mounting ring from the hook.

### Detailed description of the disclosure

In the following description, the equivalent reference numerals are used in different embodiments to denote equivalent or similar features.

Unless defined otherwise, all technical and scientific terms used in this specification have the same meaning as is commonly understood by the reader skilled in the art to which the claimed subject matter belongs. It is to be understood that the foregoing summary of the disclosure and the following examples are exemplary and explanatory only and are not restrictive of any subject matter claimed.

In this specification, the use of the singular includes the plural unless the context clearly dictates otherwise. In this application, the use of "or" means "and/or" unless stated otherwise. Furthermore, use of the term "including" as well as other forms, such as "include", "includes," and "included," is not limiting.

As used herein, ranges and amounts can be expressed as "about" a particular value or range. About also includes the exact amount. For example, "about 5 mm" means "about 5 mm" and also "5 mm." Generally, the term "about" includes an amount that would be expected to be within experimental error. The term "about" includes values that are within 10% less to 10% greater of the value provided. For example, "about 50%" means "between 45% and 55%." Also, by way of example, "about 30" means "between 27 and 33."

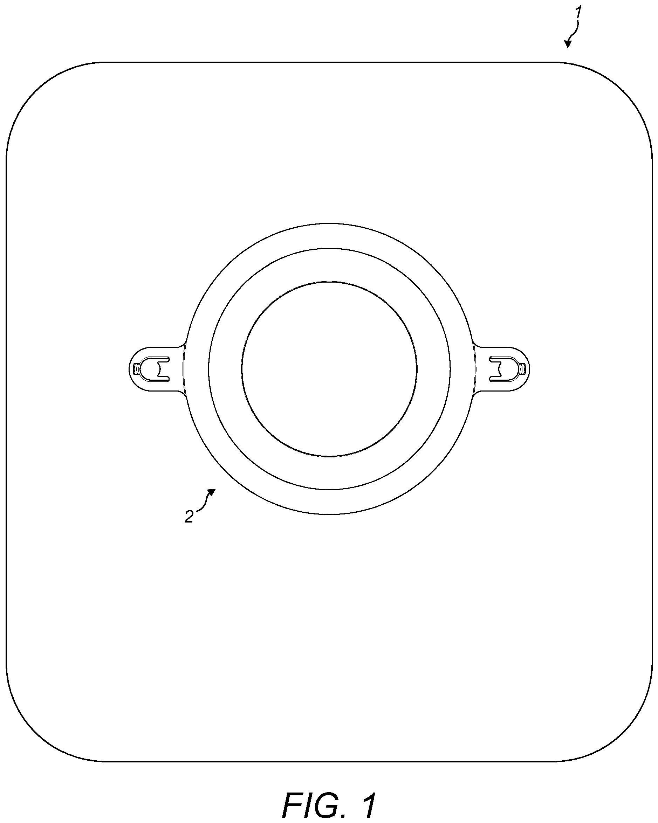

Figure 1 illustrates a schematic representation of an ostomy appliance 1 that comprises a wafer 2. The ostomy appliance 1 of Figure 1 is shown, by way of example only, as a closed appliance. The ostomy appliance 1 may generally comprise an inner wall and an outer which may be joined together to define a cavity for containing a stomal output. The inner wall may be provided with a stomal inlet for receiving the stomal output into the cavity. The stomal inlet may be an aperture that is cut out of the inner wall. The ostomy appliance 1 of Figure 1 is shown schematically as a one-piece appliance wherein the wafer 2 is permanently attached to the ostomy appliance 1, to the extent that the wafer 2 cannot easily be separated without risk of damaging the ostomy appliance 1. However, it is to be understood that the belt mount of the present disclosure may be used with a wide range of ostomy appliances and wafers and its use is not limited to those ostomy appliances and wafers shown in the illustrated examples. Further, the general design and features of an ostomy appliance 1 will be well known to the person skilled in the art and therefore will not be described further except where necessary to explain the features of the belt mount of the present disclosure.

In the following description the present disclosure will be described, by way of example only, with one or more mounting rings 11 of the belt mount 5 forming part of the wafer 2 and which are configured to be attached to one or more hooks forming part of a belt 40. However, it will be appreciated that this configuration may be reversed such that the one or more hooks are formed as part of the wafer 2 and one or more mounting rings 11 are formed as part of the belt 40.

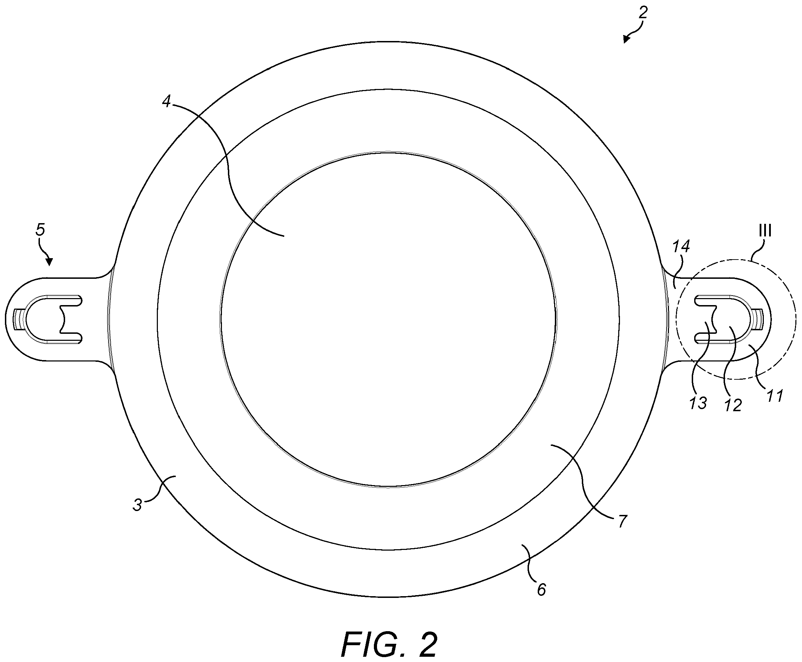

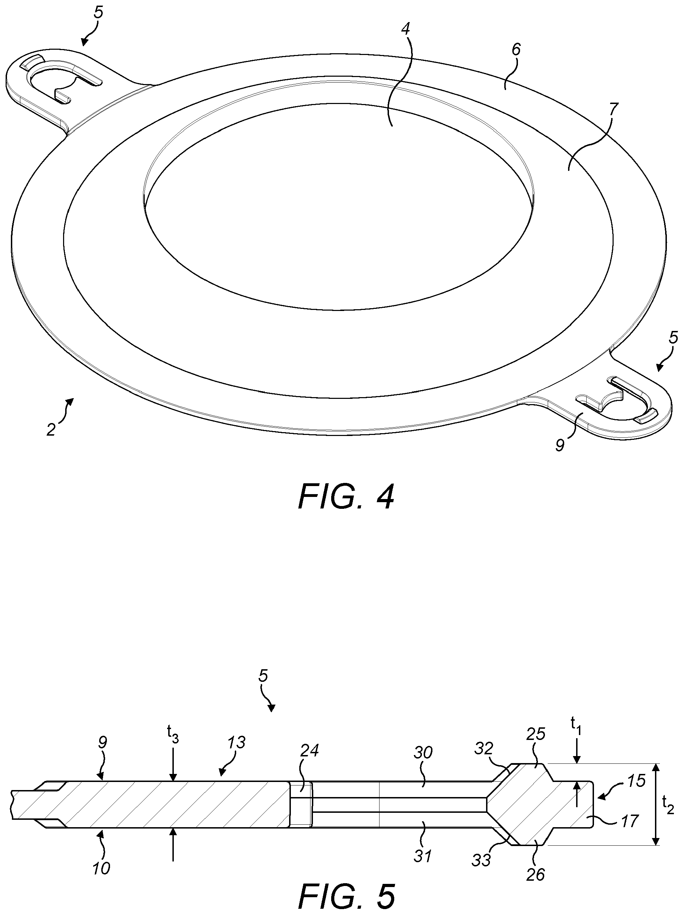

Figures 2 to 4 illustrate an example of the wafer 2 comprising a belt mount 5. The wafer 2 comprises a body 3 that may be annular in shape. The body 3 may surround an aperture 4. The aperture 4 may be aligned in use with the stomal inlet in the inner wall of the ostomy appliance 1. As best seen in Figure 4, the body 3 may comprise a peripheral region 6 which may be flat and a convex region 7 which may extend upwards out of the plane of the peripheral region 6. An inner edge of the convex region 7 may define the aperture 4.

The peripheral region 6 may have a width of 6 to 18 mm and a thickness of 0.75 to 1.00 mm, optionally 0.80 mm. The convex region 7 may have a thickness of 1 to 3 mm, optionally 1.40 mm, 2.00 mm, or 2.65 mm. The aperture 4 may have a diameter of 25 to 60 mm.

The wafer 2 may be provided with the belt mount 5. The belt mount 5 may be integrally formed as part of the wafer 2 or may be one or more separate components which are joined to a main part of the wafer 2. The belt mount 5 may comprise one or more mounting rings 11 (which may also be referred to as 'tabs'). The belt mount 5 may comprise two, three or more mounting rings 11. The one or more mounting rings 11 may be arranged at different locations around a circumference of the body 3. Two of the one or more mounting rings 11 may be disposed opposite one another, for example 180° apart around the circumference of the body 3. Where a third mounting ring 11 is present this may be located at an acute angle to one of the other two mounting rings 11 and at an obtuse angle to the other of the other two mounting rings 11.

Where two or more mounting rings 11 are present they may have the same shape, size and configuration as each other.

The or each mounting ring 11 may protrude from the body 3. The or each mounting ring 11 may protrude outwards from the body 3. The or each mounting ring 11 may be oriented generally along a radial line that extends from a centre of the aperture 4. The or each mounting ring 11 may generally lie in the plane of the peripheral region 6 as best seen in Figure 4.

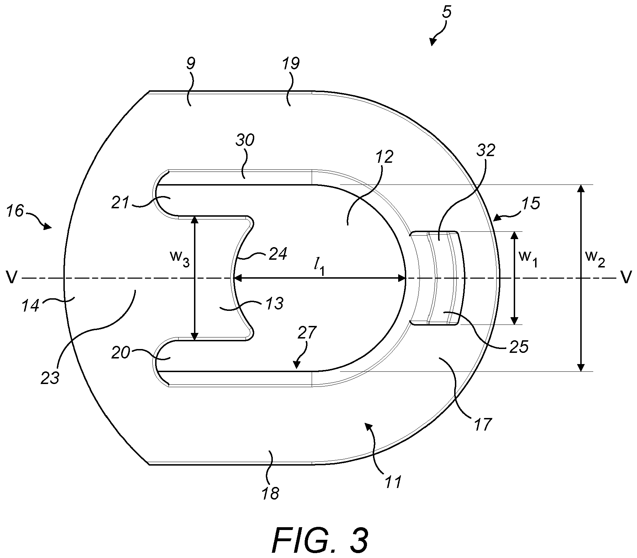

As shown in Figures 2 and 3, the or each mounting ring 11 may comprise a distal end 15 furthest from the body 3 and a proximal end 16 closest to the body 3. The proximal end 16 may directly join or merge with the peripheral region 6 of the body 3. A support region 14 may be defined at or near the proximal end 16 which supports a remainder of the mounting ring 11 which may extend away from the support region 14 in a cantilevered manner.

The or each mounting ring 11 may comprise a first leg 18 and a second leg 19 which may extend from the proximal end 16 towards the distal end 15. A proximal end of each of the first leg 18 and the second leg 19 may be joined to or merge with the support region 14. A distal end of each of the first leg 18 and the second leg 19 may be joined to or merge with opposite ends of an end portion 17 of the mounting ring 11. The first leg 18 and the second leg 19 may both be straight. The end portion 17 may be rounded, optionally semi-circular in shape. The first leg 18, second leg 19 and end portion 17 may together define a mounting ring 11 that is D-shaped as most clearly seen in Figure 3. The or each mounting ring 11 may be a unitary part, optionally a unitary moulded part. Optionally, the body 3 and all of the one or more mounting rings 11 of the wafer may be a unitary part, optionally a unitary moulded part.

The belt mount 5 may be formed from a flexible material. Optionally the belt mount 5 is formed from a material that is resilient. Optionally the whole of the wafer 2 including the belt mount 5 is formed from a flexible and/or resilient material. For example, the material of at least the belt mount 5 may be a polymer or co-polymer. In one example the material may be ethylene-vinyl acetate (EVA).

The first leg 18, second leg 19 and end portion 17 may together surround and at least partially define a mounting aperture 12. The mounting aperture 12 may be elongated in a radial direction. A width w₁ of the mounting aperture 12 may be 4 to 8 mm, optionally 6 mm.

The or each mounting ring 11 may further comprise a tongue 13 as shown in Figure 3. The tongue 13 may extend from a support portion 23 into the mounting aperture 12. The support portion 23 may be part of or adjacent the support region 14. The length of the tongue 13 may be less than a length (in the radial direction) of the mounting aperture 12 such that a distal end of the tongue 13 does not reach into contact with the end portion 17 of the mounting ring 11. As such, due to the presence of the tongue 13, an available opening of the mounting aperture 12 may have a length l₁ as shown in Figure 3.

Optionally, as shown in the example of Figure 3, the length of the tongue 13 may be less than 50% of the length of the mounting aperture 12. The distal end of the tongue 13 may comprise a concave face 24 as seen in Figure 3.

A width of the tongue 13 may be less than a width of the mounting aperture 12 between the first leg 18 and the second leg 19. A width w₃ of the tongue 13 may be 2 to 6 mm, optionally 4 mm. Thus, a first clearance 20 and a second clearance 21 may be formed between either side of the tongue 13 and the first leg 18 and the second leg 19. Each clearance 20, 21 may have a width of 1 mm.

The tongue 13 may be flexible. In particular, the tongue 13 may be flexed about the support portion 23 such that the distal end of the tongue is moved out of the plane of the mounting ring 11 as will be described further below.

As seen in Figures 4 and 5, the or each mounting ring 11 may be generally thin. The or each mounting ring 11 may have the same thickness or a greater thickness than that of the peripheral region 6. The general thickness of the mounting ring 11 maybe 1.0 to 1.5 mm, optionally 1.3 mm.

A first face 9 of the or each mounting ring 11 and a second face 10 of the or each mounting ring 11 may be parallel to one another over a majority of their area. The first leg 18, second leg 19 and tongue 13 may have the same thickness as each other. A thickness t₃ of the tongue 13 may be 1.0 to 1.5 mm, optionally 1.3 mm. The thickness of the end portion 17 of the or each mounting ring 11 may be the same as that of the tongue 13 except for the provision of one or more protrusions 25, 26 as will be described further below.

At least a portion of an inner edge 27 of the or each mounting ring 11 may comprise a chamfered edge. A chamfer may be provided at an inner edge of the first leg 18, the second leg 19 and/or the end portion 17. A chamfer may be provided at the first face 9 and/or the second face 10. In the example of Figure 5, a first chamfer 30 is provided at the first face 9 of the inner edge 27 around the full extent of the first leg 18, the second leg 19 and the end portion 17. The first chamfer 30 may extend around a U-shape. Likewise a second chamfer 31 is provided at the second face 10 of the inner edge 27 around the full extent of the first leg 18, the second leg 19 and the end portion 17. The second chamfer 31 may extend around a U-shape.

The or each mounting ring 11 may further comprise at least one protrusion 25, 26 that extends from at least one of the first face 9 and the second face 10 of the or each mounting ring 11. The or each mounting ring 11 may comprise only a first protrusion 25 that extends from the first face 9 but no protrusion on the second face 10. Alternatively, the or each mounting ring 11 may comprise only a second protrusion 26 that extends from the second face 10 but no protrusion on the first face 9. Alternatively, the or each mounting ring 11 may comprise both a first protrusion 25 that extends from the first face 9 and a second protrusion 26 that extends from the second face 10, as most clearly seen in the example of Figure 5. In the later example, optionally the first protrusion 25 and second protrusion 26 are configured as mirror-images of each as reflected in the plane of the mounting ring 11. Also optionally the first protrusion 25 and second protrusion 26 may be aligned with one another about a circumference of the mounting ring 11.

As best seen in Figure 5, the thickness t₁ of the first protrusion 25 and or the second protrusion 26 may be 0.4 to 0.6 mm, optionally 0.5 mm. The total thickness t₂ of the mounting ring 11 at the location of the first protrusion 25 and or second protrusion 26 may be 1.8 to 2.7 mm, optionally 2.3 mm

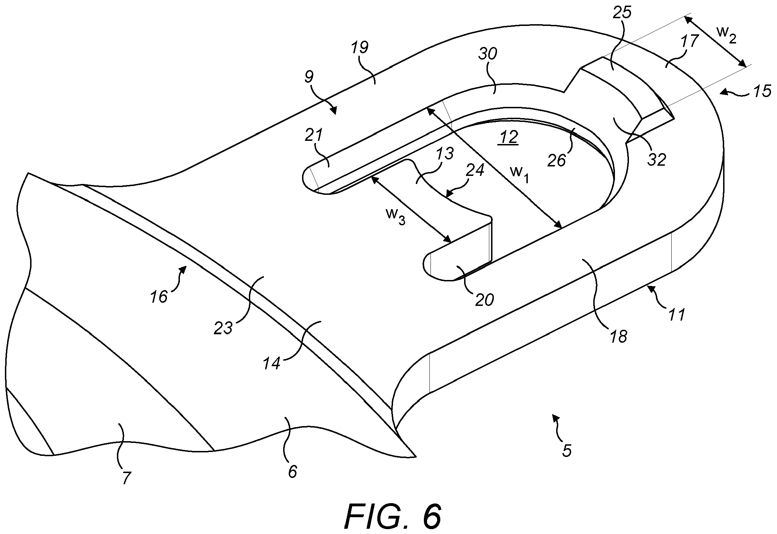

As most clearly seen in Figure 6, the first protrusion 25 and or the second protrusion 26 may be located at the distal end 15 of the mounting ring 11 and/or at the distal end of the mounting aperture 12. Optionally, the first protrusion 25 and or the second protrusion 26 may be located opposite the tongue 13. The first protrusion 25 and or the second protrusion 26 may be located adjacent to the inner edge 27.

The first protrusion 25 and or the second protrusion 26 may each comprise a ramped surface 32, 33 facing towards the mounting aperture 12. The first protrusion 25 may comprise a first ramped surface 32 that faces towards the mounting aperture 12. The first ramped surface 32 may be formed as a smooth continuation of the first chamfer 30 as shown in Figure 6. Likewise, the second protrusion 26 may comprise a second ramped surface 33 that faces towards the mounting aperture 12. The second ramped surface 33 may be formed as a smooth continuation of the second chamfer 31.

The first protrusion 25 and or the second protrusion 26 may be formed from a compressible material, optionally a resilient material. The material of the first protrusion 25 and or the second protrusion 26 may be the same as that of the remainder of the mounting ring 11. Optionally the first protrusion 25 and or the second protrusion 26 are integrally formed with a remainder of the mounting ring 11, for example as an integrally moulded part.

The first protrusion 25 and or the second protrusion 26 may have a width no greater than a width of the tongue 13. The first protrusion 25 and or the second protrusion 26 may have a width that is less than a width of the tongue 13. A width w₂ of the first protrusion 25 and or the second protrusion 26 may be 2.5 to 3.5 mm, optionally 3.0 mm.

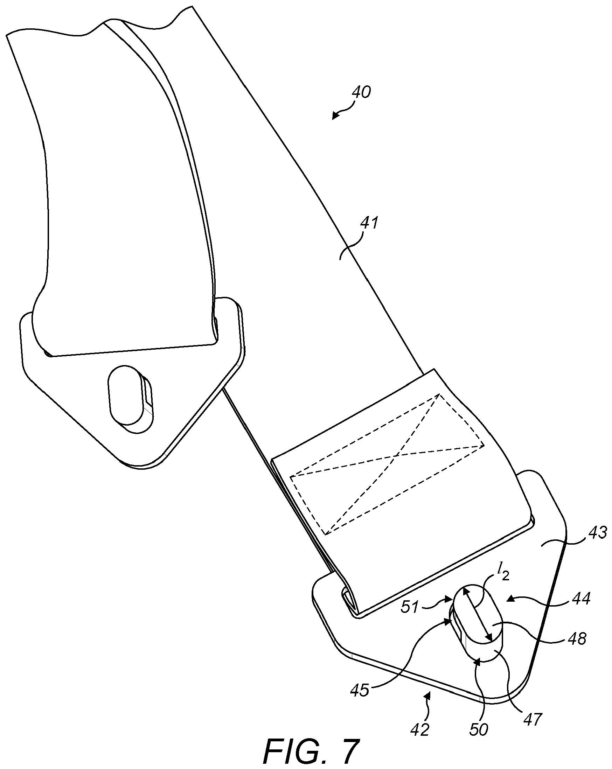

Figure 7 illustrates a belt 40 which comprises a strap 41 having a hook 42 at each end. Each hook 42 may comprise a baseplate 43 and a cantilevered hook element 44. The cantilevered hook element 44 may comprise a support portion 50 that may have a convex face 47 and a projecting portion 51 that extends from the support portion 50. The projecting portion 51 may have a length l₂. Optionally the length l₂ of the projecting portion 51 is longer than the length l₁ of the available opening of the mounting aperture 12.

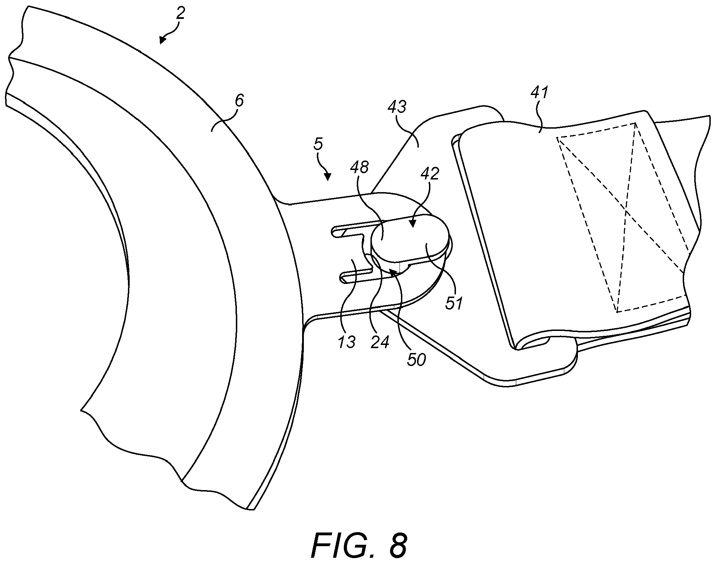

A mouth 45 of the hook 42 may be defined between the baseplate 43 and the cantilevered hook element 44, in particular between the baseplate 43 and the projecting portion of the cantilevered hook element 44. The hook 42, as shown in Figures 7 and 8, is 'reverse-directed' in that the mouth 45 of the hook 42 faces away from the wafer 2 when attached to the belt mount 5 as seen in Figure 8.

The baseplate 43 may comprise an aperture 46. Optionally the aperture 46 is in register with the cantilevered hook element 44, e.g. located directly below the cantilevered hook element 44. Optionally the aperture 46 is of the same or substantially the same shape and size (in plan view) as the cantilevered hook element 44. An underside of the cantilevered hook element 44 may comprises a recess.

In use, the belt mount 5 may be used for attaching the belt 40 to the wafer 2 of the ostomy appliance 1. To do so for the illustrated example, the ostomate or user may attach a first hook 42 of belt 40 to a first mounting ring 11 of the belt mount 5 of the wafer 2, pass the strap 41 around the torso, limb or other body part of the ostomate, and then attach a second hook 42 of the belt 40 to a second mounting ring 11 of the belt mount 5 of the wafer 2. The strap 41 may then be tightened as required.

The or each mounting ring 11 may be attached to the hook 42 by inserting a portion of the mounting ring 11 into the mouth 45 of the hook 42 to achieve the state shown in Figure 8.

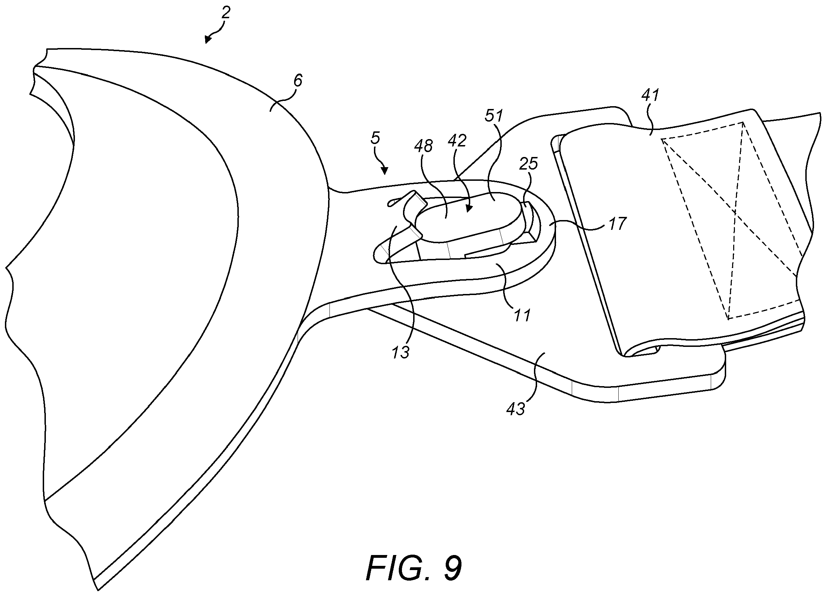

Insertion may be achieved by inserting the portion of the mounting ring 11 into the mouth 45 of the hook 42 and pulling the hook 42 and the mounting ring 11 away from each other, due to the hook 42 being reverse-directed. Optionally the portion of the mounting ring 11 that is inserted is the end portion 17. As shown in Figure 9, to insert the end portion 17 of the mounting ring 11, the wafer 2 may be angled relative to the baseplate 43 and the mounting ring 11 may be flexed.

The size and shape of the mounting ring 11, for example the thickness of one or more portions of the mounting ring 11, may be configured relative to the size and shape of the hook 42 to ensure that there is contact between the mounting ring 11 and the hook 42 when inserting the portion of the mounting ring 11 into the mouth 45 of the hook 42.

The thickness t₃ of the first leg 18 and the second leg 19 may be less than a clearance of the mouth 45. However, the thickness t₁ of the first protrusion 25 and or the second protrusion 26 may result in the total thickness t₂ of the mounting ring 11 at the location of the first protrusion 25 and or second protrusion 26 being greater than the clearance of the mouth 45. In this way, the first protrusion 25 and or the second protrusion 26 may be configured to provide a resistance to insertion of the end portion 17 of the mounting ring 11 into the mouth 45 of the hook 42. In particular, during insertion, the first protrusion 25 that extends from the first face 9 may contact the projecting portion 51 of the cantilevered hook element 44. Additionally or alternatively, the second protrusion 26 that extends from the second face 10 may contact the baseplate 43 at or near the aperture 46.

The hook 42 may be engaged with the mounting ring 11 from either side, e.g. with the hook element 44 entering the mounting aperture 12 from either the side of the first face 9 or from the side of the second face 10.

The first chamfer 30 and or the second chamfer 31 may ease insertion of the mounting ring 11 into the hook 42.

The projecting portion 51 may make first contact with the first ramped surface 32 of the first protrusion 25. The angle of the first ramped surface 32 may tend to urge the projecting portion 51 to flex upwards. Alternatively, the projecting portion 51 may make first contact with the second ramped surface 33 of the second protrusion 26 when the hook element 44 is attached from the other side of the mounting ring 11. The angle of the second ramped surface 33 may tend to urge the projecting portion 51 to flex upwards

The first protrusion 25 and or the second protrusion 26 may have a width no greater than a width of the hook 42.

As best seen in Figure 9, during insertion the tongue 13 may make contact with the hook element 42 as the end portion 17 is contacted with the projecting portion 51. Optionally, the tongue 13 may be configured to flex and ride over a back 48 of the projecting portion 51 on insertion of the end portion 17 of the mounting ring 11 into the mouth 45 of the hook 42. Optionally this may be due to the length l₂ of the projecting portion 51 being longer than the length l₁ of the available opening of the mounting aperture 12.

Overcoming the resistance to insertion provided by the first protrusion 25 and or the second protrusion 26 may be achieved by the ostomate or user pulling harder on the strap 41. Passage of the end portion 17 into the hook 42 may be accommodated by one or more of compression of the material of the first protrusion 25 and or the second protrusion 26 and deflection upwards of the projecting portion 51 of the cantilevered hook element 44. Optionally both the mounting ring 11 and the hook 42 are formed of resilient materials such that the first protrusion 25 and or the second protrusion 26 and the cantilevered hook element 44 recover their shapes after insertion. Optionally, at the same time the tongue 13 may complete its passage over the back 48 of the projecting portion 51.

Advantageously, the configuration of the belt mount 5 may produce at least one of a tactile feedback and an audible feedback signalling successful insertion of the end portion 17 of the mounting ring 11 into the mouth 45 of the hook 42. The tactile and or audible feedback may result from one or more of the tongue 13 coming out of contact with the hook element 42, the tongue 13 subsequently hitting the baseplate 43 as it resiliently recovers its shape, and the end portion 17 'popping through' the mouth 45 of the hook element and hitting the support portion 50 of the hook element 42.

In the attached position, the second protrusion 26 may be received in the aperture 46 of the baseplate 43. The first protrusion may be received in the recess on the underside of the cantilevered hook element 44.

In the attached position, the concave face 24 of tongue 13 may also be separated from the convex face 47 of the hook element 44 by a small gap. This may allow a small amount of play between the hook 42 and the belt mount 5. However, optionally the gap is small enough that if the hook 42 and belt mount 5 are simply pulled part the concave face 24 will contact the convex face 47 while the second protrusion 26 is still received in the aperture 46 of the baseplate 43. Thus, abutment between the concave face 24 and the convex face 47 may act to hinder detachment of the belt mount 5 from the hook 42 by means of a linear compressive force.

The concave face 24 may be shaped to cooperate with the convex face 47 to guide the hook 42 in limited rotation within the belt mount 5. For example, a width of the cantilevered hook element 44 may be less than the width w₁ of the mounting aperture 12 so as to accommodate limited rotation of the hook 42 and the baseplate 43 about an axis extending perpendicularly to a plane of the mounting ring 11. The limited rotation of the hook 42 and the baseplate 43 may be guided by contact between the concave face 24 of the tongue 13 and the convex face 47 of the cantilevered hook element 44. Optionally, the limited rotation of the hook 42 and the baseplate 43 may be constrained by contact between the cantilevered hook element 44 and the mounting ring 11. For example the rotation may be constrained by contact of the support portion 50 with the first leg 18 and or second leg 19 of the mounting ring 11.

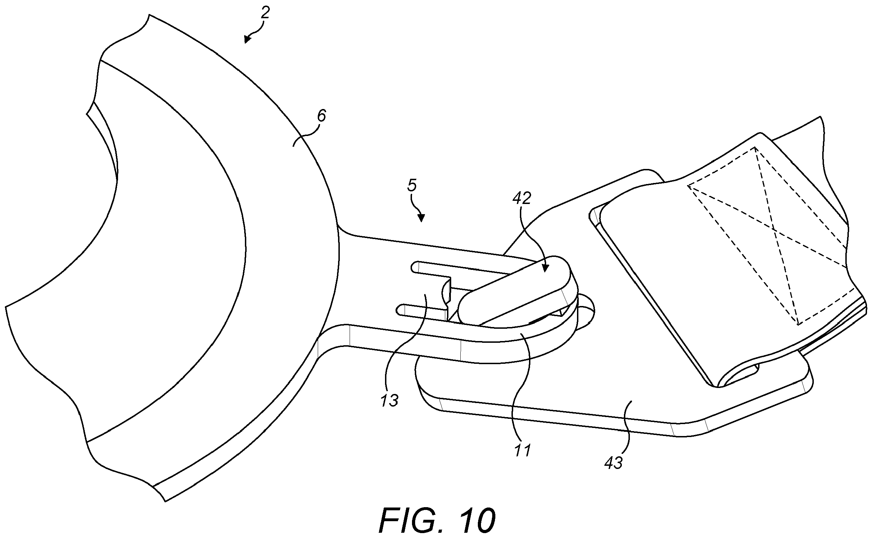

Detachment of the hook 42 from the mounting ring 11 is shown in Figure 10. Optionally the wafer 2 and the hook 42 are angled relative to one another, for example by rotating the wafer 2 and mounting ring 11 around an effective pivot point located at the distal end 15 of the mounting ring 11 such that the tongue 13 is raised clear of the cantilevered hook element 44. The hook 42 and the mounting ring 11 may thereafter be separated from one another by pushing the two components together which causes the end portion 17 of the mounting ring 11 to be pushed out of the mouth 45. Similar to the process of insertion, the movement of detachment may be accommodated by one or more of compression of the material of the first protrusion 25 and or the second protrusion 26 and deflection upwards of the projecting portion 51 of the cantilevered hook element 44.

### Examples

The following table presents example configurations of a wafer 2 comprising a belt mount 5 according to the present disclosure. These examples are not intended to be limiting on the present disclosure in any way or to limit the scope of the appended claims. Rather, the examples are provided to aid a better understanding of the present disclosure.

The examples refer to features described in further detail elsewhere in the present disclosure. The skilled reader will understand that reference should be made to said further description where necessary for a fuller understanding of the examples. Where said further description refers to optional characteristics of said features then the skilled reader will understand that the following examples may optionally also include one or more of said optional characteristics.

In the following examples, each mounting ring 11 has a configuration as shown in Figure 3 and comprising:
- t₁ = 0.5 mm
- t₂ = 2.3 mm
- t₃ = 1.3 mm
- a thickness of peripheral region 6 = 0.8 mm
- w₁ = 6 mm
- w₂ = 3 mm
- w₃ = 4 mm
- A first protrusion 25 and a second protrusion 26.

In the following examples, each wafer 2 has two opposed mounting rings 11. As an alternative, the examples may also be provided with a third mounting ring 11 of the same shape and configuration.

| Example No. | Maximum dimension between distal ends of mounting rings (mm) | Diameter of body 3 (mm) | Width of peripheral region 6 (mm) | Diameter of aperture 4 (mm) | Thickness of convex region 7 (mm) |
|---|---|---|---|---|---|
| 1 | 115 | 89 | 7.5 | 58 | 2.00 |
| 2 | 110 | 80 | 6.5 | 48 | 2.65 |
| 3 | 110 | 80 | 11.5 | 38 | 2.65 |
| 4 | 110 | 80 | 16.5 | 28 | 2.65 |
| 5 | 115 | 89 | 8 | 58 | 1.40 |
| 6 | 110 | 80 | 8 | 48 | 1.40 |
| 7 | 110 | 80 | 8 | 38 | 1.40 |
| 8 | 110 | 80 | 8 | 28 | 1.40 |

## Claims

1. A system comprising a hook (42) and a belt mount (5) for attaching a belt (40) to a wafer (2) of an ostomy appliance (1), the belt mount (5) comprising a mounting ring (11) configured to be attached to said hook (42) by inserting a portion of the mounting ring (11) into a mouth (45) of the hook (42) and pulling the mounting ring (11) and hook (42) away from each other;
the mounting ring (11) at least partially surrounding a mounting aperture (12) which accommodates at least a portion of the hook (42) upon successful insertion of the portion of the mounting ring (11) into the mouth (45) of the hook (42);
**characterized in that** the belt mount (5) further comprising a tongue (13) that extends into the mounting aperture (12) for contacting the hook (42) if the mounting ring (11) and the hook (42) are subsequently pushed towards each other so as to resist detachment of the mounting ring (11) from the hook (42),
wherein the hook (42) comprises a baseplate (43) and a cantilevered hook element (44), wherein a mouth (45) of the hook (42) is defined between the baseplate (43) and the cantilevered hook element (44).

2. The system of claim 1, wherein the tongue (13) extends in a plane that is parallel with a face of the mounting ring (11).

3. The system of claim 1 or claim 2, wherein the tongue (13) extends from a proximal end of the mounting aperture (12).

4. The system of any preceding claim, wherein the tongue (13) comprises a concave end that is contactable with the hook (42) if the mounting ring (11) and the hook (42) are pushed towards each other.

5. The system of any preceding claim, further comprising a clearance either side of tongue (13) and in between the tongue (13) and the mounting ring (11), and the tongue (13) is cantilevered from a supporting portion of the belt mount (5).

6. The system of any preceding claim, wherein the tongue (13) is flexible.

7. The system of any preceding claim, wherein the tongue (13) is configured to flex and ride over a back portion of the hook (42) on insertion of the portion of the mounting ring (11) into the mouth of the hook (42).

8. The system of any preceding claim, further comprising at least one protrusion (25, 26) that extends from at least one of a first face (9) and a second face (10) of the mounting ring (11) to contact the hook (42) when inserting the portion of the mounting ring (11) into the mouth of the hook (42), wherein the at least one protrusion (25, 26) is located at a distal end of the mounting ring (11) opposite the tongue (13).

9. The system of claim 8, wherein the at least one protrusion (25, 26) has a width no greater than a width of the tongue (13).

10. The system of any preceding claim, wherein a width of the cantilevered hook element (44) is less than a width of the mounting aperture (12) so as to accommodate limited rotation of the hook (42) and the baseplate (43) about an axis extending perpendicularly to a plane of the mounting ring (11).

11. The system of claim 10, wherein the limited rotation of the hook (42) and the baseplate (43) is guided by contact between a concave end of the tongue (13) and a convex face of the cantilevered hook element (44); and
wherein the limited rotation of the hook (42) and the baseplate (43) is constrained by contact between the cantilevered hook element (43) and the mounting ring (11).

12. The system of any preceding claim, wherein the tongue (13) is configured to contact the baseplate (43) to cause at least one of a tactile feedback and an audible feedback as the tongue (13) recovers its shape upon successful insertion of the portion of the mounting ring (11) into the mouth of the hook (42).

## Patentansprüche

1. System umfassend einen Haken (42) und eine Gurthalterung (5) zum Befestigen eines Gurtes (40) an einem Wafer (2) einer Ostomievorrichtung (1), wobei die Gurthalterung (5) einen Halterungsring (11) umfasst, der ausgebildet ist, um an dem Haken (42) befestigt zu werden, indem ein Teil des Halterungsrings (11) in eine Mündung (45) des Hakens (42) eingeführt wird und der Halterungsring (11) und der Haken (42) voneinander weggezogen werden;
wobei der Halterungsring (11) mindestens teilweise eine Halterungsöffnung (12) umgibt, die mindestens einen Teil des Hakens (42) nach erfolgreichem Einführen des Teils des Halterungsrings (11) in die Mündung (45) des Hakens (42) aufnimmt;
**dadurch gekennzeichnet, dass** die Gurthalterung (5) ferner eine Zunge (13) umfasst, die sich in die Halterungsöffnung (12) erstreckt, um mit dem Haken (42) in Kontakt zu kommen, wenn der Halterungsring (11) und der Haken (42) sodann aufeinander zu geschoben werden, um einem Lösen des Halterungsrings (11) vom Haken (42) zu widerstehen,
wobei der Haken (42) eine Grundplatte (43) und ein auskragendes Hakenelement (44) umfasst, wobei eine Mündung (45) des Hakens (42) zwischen der Grundplatte (43) und dem auskragenden Hakenelement (44) definiert ist.

2. System gemäß Anspruch 1, wobei sich die Zunge (13) in einer Ebene erstreckt, die parallel zu einer Fläche des Halterungsrings (11) ist.

3. System gemäß Anspruch 1 oder Anspruch 2, wobei sich die Zunge (13) von einem proximalen Ende der Halterungsöffnung (12) aus erstreckt.

4. System gemäß einem der vorhergehenden Ansprüche, wobei die Zunge (13) ein konkaves Ende umfasst, das mit dem Haken (42) in Kontakt kommen kann, wenn der Halterungsring (11) und der Haken (42) aufeinander zu geschoben werden.

5. System gemäß einem der vorhergehenden Ansprüche ferner umfassend einen Freiraum auf beiden Seiten der Zunge (13) und zwischen der Zunge (13) und dem Halterungsring (11), und wobei die Zunge (13) von einem Stützteil der Gurthalterung (5) auskragend ist.

6. System gemäß einem vorhergehenden Anspruch, wobei die Zunge (13) flexibel ist.

7. System gemäß einem der vorhergehenden Ansprüche, wobei die Zunge (13) so ausgebildet ist, dass sie sich beim Einführen des Teils des Halterungsrings (11) in die Mündung des Hakens (42) biegt und über einen hinteren Teil des Hakens (42) fährt.

8. System gemäß einem der vorhergehenden Ansprüche ferner umfassend mindestens eine Erhebung (25, 26), die sich von mindestens einer ersten Fläche (9) und einer zweiten Fläche (10) des Halterungsrings (11) erstreckt, um mit dem Haken (42) in Kontakt zu kommen, wenn der Teil des Halterungsrings (11) in die Mündung des Hakens (42) eingeführt wird, wobei die mindestens eine Erhebung (25, 26) an einem distalen Ende des Halterungsrings (11) gegenüber der Zunge (13) angeordnet ist.

9. System gemäß Anspruch 8, wobei die mindestens eine Erhebung (25, 26) eine Breite aufweist, die nicht größer ist als eine Breite der Zunge (13).

10. System gemäß einem der vorhergehenden Ansprüche, wobei eine Breite des auskragenden Hakenelements (44) geringer ist als eine Breite der Halterungsöffnung (12), um eine begrenzte Rotation des Hakens (42) und der Grundplatte (43) um eine Achse, die sich senkrecht zu einer Ebene des Halterungsrings (11) erstreckt, zu ermöglichen.

11. System gemäß Anspruch 10, wobei die begrenzte Rotation des Hakens (42) und der Grundplatte (43) durch den Kontakt zwischen einem konkaven Ende der Zunge (13) und einer konvexen Fläche des auskragenden Hakenelements (44) geführt wird; und wobei die begrenzte Rotation des Hakens (42) und der Grundplatte (43) durch den Kontakt zwischen dem auskragenden Hakenelement (43) und dem Halterungsring (11) eingeschränkt ist.

12. System gemäß einem der vorhergehenden Ansprüche, wobei die Zunge (13) ausgebildet ist, um mit der Grundplatte (43) in Kontakt zu kommen, um mindestens eine taktile Rückmeldung und eine akustische Rückmeldung zu bewirken, wenn die Zunge (13) nach erfolgreichem Einsetzen des Teils des Halterungsrings (11) in die Mündung des Hakens (42) ihre Form wiedererlangt.

## Revendications

1. Système comprenant un crochet (42) et un support (5) de ceinture pour fixer une ceinture (40) à une rondelle (2) d'un appareillage (1) de stomie, le support (5) de ceinture comprenant un anneau (11) de montage configuré pour être fixé audit crochet (42) par insertion d'une partie de l'anneau (11) de montage dans une bouche (45) du crochet (42) et éloignement de l'anneau (11) de montage et du crochet (42) l'un de l'autre en les tirant ;
l'anneau (11) de montage entourant au moins en partie une ouverture (12) de montage, dans laquelle est logée au moins une partie du crochet (42) après insertion réussie de la partie de l'anneau (11) de montage dans la bouche (45) du crochet (42) ;
**caractérisé en ce que** le support (5) de ceinture comprend en outre une languette (13), qui s'étend dans l'ouverture (12) de montage pour être au contact du crochet (42), si l'anneau (11) de montage et le crochet (42) sont ensuite poussés l'un vers l'autre, de manière à empêcher que l'anneau (11) de montage ne se détache du crochet (42),
dans lequel le crochet (42) comprend une plaque (43) de base et un élément (44) de crochet en porte à faux, une bouche (45) du crochet (42) étant définie entre la plaque (43) de base et l'élément (44) de crochet en porte à faux.

2. Système suivant la revendication 1, dans lequel la languette (13) s'étend dans un plan, qui est parallèle à une face de l'anneau (11) de montage.

3. Système suivant la revendication 1 ou la revendication 2, dans lequel la languette (13) s'étend à partir d'une extrémité proximale de l'ouverture (12) de montage.

4. Système suivant n'importe quelle revendication précédente, dans lequel la languette (13) comprend une extrémité concave, qui peut être mise au contact du crochet (42) si l'anneau (11) de montage et le crochet (42) sont poussés l'un vers l'autre.

5. Système suivant n'importe quelle revendication précédente, comprenant en outre un jeu de chaque côté de la languette (13) et entre la languette (13) et l'anneau (11) de montage, et la languette (13) est en porte à faux à partir d'une partie de support du support (5) de ceinture.

6. Système suivant n'importe quelle revendication précédente, dans lequel la languette (13) est souple.

7. Système suivant n'importe quelle revendication précédente, dans lequel la languette (13) est configurée pour fléchir et venir sur une partie arrière du crochet (42) à l'insertion de la partie de l'anneau (11) de montage dans la bouche du crochet (42).

8. Système suivant n'importe quelle revendication précédente, comprenant en outre au moins une saillie (25, 26), qui s'étend à partir d'au moins une première face (9) et d'une deuxième face (10) de l'anneau (11) de montage pour être au contact du crochet (42) lors de l'insertion de la partie de l'anneau (11) de montage dans la bouche du crochet (42), la au moins une saillie (25, 26) étant placée à une extrémité distale de l'anneau (11) de montage opposé à la languette (13).

9. Système suivant la revendication 8, dans lequel la au moins une saillie (25, 26) a une largeur, qui n'est pas plus grande qu'une largeur de la languette (13).

10. Système suivant n'importe quelle revendication précédente, dans lequel une largeur de l'élément (44) de crochet en porte à faux est plus petite qu'une largeur de l'ouverture (12) de montage de manière à permettre une rotation limitée du crochet (42) et de la plaque (43) de base autour d'un axe s'étendant perpendiculairement à un plan de l'anneau (11) de montage.

11. Système suivant la revendication 10, dans lequel la rotation limitée du crochet (42) et de la plaque (43) de montage est guidée par contact entre une extrémité concave de la languette (13) et une face convexe de l'élément (44) de crochet en porte à faux ; et
dans lequel la rotation limitée du crochet (42) et de la plaque (43) de base est contrainte par contact entre l'élément (43) de crochet en porte à faux et l'anneau (11) de montage.

12. Système suivant n'importe quelle revendication précédente, dans lequel la languette (13) est configurée pour être en contact avec la plaque (43) de base pour provoquer au moins l'une d'une réaction tactile et d'une réaction audible, alors que la languette (13) retrouve sa forme après insertion réussie de la partie de l'anneau (11) de montage dans la bouche du crochet (42).
